Europäisches Patentamt

European Patent Office

Office européen des brevets

(19)

(11) **EP 1 051 155 B1**

(12) **EUROPEAN PATENT SPECIFICATION**

(45) Date of publication and mention
of the grant of the patent:
**26.06.2002 Bulletin 2002/26**

(21) Application number: **99904823.4**

(22) Date of filing: **28.01.1999**

(51) Int Cl.[7]: **A61K 9/00**

(86) International application number:
**PCT/EP99/00555**

(87) International publication number:
**WO 99/38492 (05.08.1999 Gazette 1999/31)**

(54) **NASAL SOLUTIONS**

NASALE LÖSUNGEN

PREPARATIONS PHARMACEUTIQUES

(84) Designated Contracting States:
**AT BE CH CY DE DK ES FI FR GB GR IE IT LI LU
MC NL PT SE**

(30) Priority: **30.01.1998 EP 98810069**

(43) Date of publication of application:
**15.11.2000 Bulletin 2000/46**

(73) Proprietor: **Novartis Consumer Health S.A.
1260 Nyon (CH)**

(72) Inventors:
• **SEIDEL, Matthias
CH-3063 Ittigen (CH)**

• **BUCKLEY, Christopher
CH-1291 Commugny (CH)**

(74) Representative: **Becker, Konrad et al
Novartis AG
Corporate Intellectual Property
Patent- und Markenabteilung
4002 Basel (CH)**

(56) References cited:
**EP-A- 0 277 462          EP-A- 0 582 259**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to pharmaceutical compositions intended for nasal administration. More specifically, it concerns liquid nasal formulations with improved moisturizing properties.

The nasal administration of active substances is a widely used method of treatment. Active substances which come into consideration are, for example, vasoconstrictors, such as xylometazoline, or antiallergic agents, such as cromoglycic acid or $H_1$ receptor antagonists, e.g. dimethindene maleate. Another group of possible active substances is e.g. corticosteroids, such as beclomethasone or fluticasone.

The indications in which a certain nasally administered drug is to be applied are known in the art. For example, vasoconstrictors are e.g. used as nasal decongestants for alleviating the typical symptoms of common cold, like running nose, obstructed nose etc., or in rhinitis or sinusitis. Antiallergic agents and corticosteroids are e.g. used in antiallergic conditions, e.g. hay fever, or in anti-asthmatic or anti-inflammatory conditions.

Nasal administration of active substances in liquid form, e.g. in the form of drops, a solution or a spray - opposite to nasal administration in gel form - is desirable inter alia because of a much better distribution of the active substances within the - partly tiny - nasal cavities and an easier handling and dosing, e.g. in pediatric or geriatric patients.

However, upon administration of liquid nasal formulations often the patients are suffering from side-effects like burning, dryness, stinging of the nasal mucosa or sneezing. One of the reasons for this is that liquids - in contrast to gels - normally do not remain in the nasal cavities for a long period of time but are washed out fast.

The present invention addresses these problems and provides liquid nasal formulations which do not only moisturize the nasal mucosa but also keep it sufficiently moisturized for a prolonged period of time. As a result, liquid nasal pharmaceutical compositions having excellent and prolonged moisturizing properties are obtained.

The invention relates to a liquid nasal pharmaceutical composition which comprises

    (a) one or more active substances suitable for nasal administration,
    (b) sorbitol;
    (c) a water-soluble $C_1$-$C_4$-alkyl-cellulose derivative;
    (d) a vehicle which is present in an amount of at least 90% (m/V) of the total composition, and which is selected from water and mixtures of water with propylene glycol, water with glycerol and water with both propylene glycol and glycerol, whereby in all said mixtures water is present in an amount of at least 95% (m/V); and
    (e) optionally one or more nasally acceptable excipients.

Liquid nasal pharmaceutical compositions are e.g. drops, solutions, sprays (nebulizers) or metered-dose sprays. Typically, they are in the form of fluid solutions, but in one embodiment of the invention they may also be present in a slightly viscous form, e.g. like a syrup. However, they all can be clearly discriminated from nasal formulations in gel form in that they - in contrast to gels - are able to form drops and can be used as sprays.

Active substances suitable for nasal administration (a) are e.g. vasoconstrictors, e.g. xylometazoline, e.g. xylometazoline hydrochloride; indanazoline, metizoline; naphazoline, e.g. naphazoline hydrochloride; fenoxazoline, e. g. fenoxazoline hydrochloride; oxymetazoline, e.g. oxymetazoline hydrochloride; tetrahydrozoline, tramazoline, tymazoline; phenylephrine, e.g. phenylephrine hydrochloride; ephedrine, e.g. d-pseudoephedrine hydrochloride; or epinephrine; or antiallergic agents, such as (1) cromoglycic acid ( = cromolyn) or a nasally acceptable salt thereof, e.g. the disodium salt ( = disodium cromoglycate), or (2) $H_1$ receptor antagonists, e.g. dimethindene or a nasally acceptable salt thereof, e.g. dimethindene maleate; acrivastine, brompheniramine, chlorpheniramine, dexchlorpheniramine, triprolidine, bromodiphenhydramine, clemastine, phenyltoloxamine, piprinhydrinate, pyrilamine, tripelennamine, cetirizine, hydroxyzine, methdilazine, promethazine, trimeprazine, azatadine, cyproheptadine, loratadine, astemizole, diphenhydramine, levocabastine or terfenadine. Examples for corticosteroids are e.g. beclomethasone, e.g. beclomethasone dipropionate, or fluticasone, e.g. fluticasone propionate. All active substances which are capable of salt formation may be present either in free form or in the form of a nasally acceptable salt. Also mixtures of more than one active substance come into consideration, e.g. a combination of a vasoconstrictor and an antiallergic agent, such as xylometazoline plus cromoglycic acid or phenylephrine plus dimethindene, or a combination of a vasoconstrictor and a corticosteroid, such as xylometazoline plus beclomethasone.

In one embodiment of the invention, the active substances used are vasoconstrictors, e.g. xylometazoline, naphazoline, fenoxazoline, oxymetazoline, tetrahydrozoline, tramazoline, phenylephrine, ephedrine or epinephrine, or any nasally acceptable salt thereof. In particular preferred are xylometazoline and oxymetazoline and any nasally acceptable salts thereof.

The concentration of the active substances is typically chosen so that a pharmaceutically, i.e. nasally, effective dose thereof can be administered easily, e.g. by a certain number of drops or by spraying once or twice.

For example, if a vasoconstrictor is used as active substance (a), it is e.g. present in an amount of from 0.005 up to 0.5%, preferably of from 0.01 up to 0.3%, and in particular of from 0.025 up to 0.2% (m/V) of the total composition.

Sorbitol (b) can be applied e.g. in solid form or as aqueous solution, e.g. as a 50-80% - in particular 70% - non-crystallizing aqueous solution.

Sorbitol (b) is e.g. present in an amount of from 0.5 up to 5%, especially of from 0.5 up to 4.5%, more especially of from 1 up to 3% and in particular of from 1.2 up to 1.6%, (m/V) of the total composition.

A water-soluble $C_1$-$C_4$-alkyl-cellulose derivative (c) is e.g. methyl cellulose or a (hydroxy or carboxy)-substituted $C_1$-$C_4$-alkyl-cellulose, e.g. hydroxypropyl methyl cellulose, hydroxyethyl cellulose, hydroxypropyl cellulose, ethyl hydroxyethyl cellulose or carboxymethyl cellulose, e.g. carboxymethyl cellulose sodium. Preferred are hydroxypropyl methyl cellulose, methyl cellulose and carboxymethyl cellulose, especially hydroxypropyl methyl cellulose and methyl cellulose, and in particular hydroxypropyl methyl cellulose.

The water-soluble $C_1$-$C_4$-alkyl-cellulose derivative (c), especially hydroxypropyl methyl cellulose, is typically applied in solid form with viscosity grades ranging of from 400 up to 15000 mPa•s, especially of from 1000 up to 6000 mPa•s.

The water-soluble $C_1$-$C_4$-alkyl-cellulose derivative (c), especially hydroxypropyl methyl cellulose, is e.g. present in an amount of from 0.1 up to 5%, preferably of from 0.2 up to 1.9%, and in particular of from 0.3 up to 1%, (m/V) of the total composition.

The amount of (c) must be adjusted so that the resulting nasal formulation remains liquid. It strongly depends on the viscosity grade of the cellulose derivative used. So while an amount of 0.5% of (c) with a viscosity grade of 4000 mPa•s may be suitable, the amount of (c) having a higher viscosity grade may have to be reduced accordingly, and vice versa.

Preferred is the use of 0.3 up to 0.7% (m/V) of hydroxypropyl methyl cellulose with a viscosity grade of from 3000 up to 5000 mPa•s.

The vehicle (d) is present in an amount of at least 90%, preferably at least 92%, more preferably at least 95%, especially at least 96% and in particular at least 97%, (m/V) of the total composition.

The vehicle (d) is preferably water. If a mixture of water with propylene glycol and/or glycerol is applied as vehicle (d), water is present in an amount of at least 95%, preferably at least 97% and especially at least 98%, (m/V) in said mixtures.

Moreover, the nasal compositions of the invention may contain usual excipients, (e), which are known in the art and include buffering agents, chelating agents, preservatives, isotonicity regulators and the like.

In a preferred embodiment of the invention, the nasal compositions of the invention contain essentially the following components as excipients (e): sodium dihydrogen phosphate dihydrate [preferred amounts of total composition: 0.3-0.7% (m/V)] and disodium phosphate dodecahydrate [preferred amounts of total composition: 0.12-0.25% (m/V)] as buffering agents, disodium edetate [preferred amounts of total composition: 0.02-0.08% (m/V)] as chelating agent, benzalkonium chloride [preferred amounts of total composition: 0.005-0.02% (m/V)] as preservative, and sodium chloride [preferred amounts of total composition: 0.20-0.60% (m/V)] as isotonicity regulator.

The nasal compositions of the invention show e.g. excellent moisturizing properties, and they are excellently accepted by test persons. A significant reduction of symptoms like burning, dryness, stinging of the nasal mucosa or sneezing is found upon administration of the compositions.

The beneficial properties of the compositions of the invention can be demonstrated e.g. in the tests described in the following references:

Leuba, D., de Ribaupierre, Y. and Kucera, P. Ion transport, ciliary activity and mechanosensitivity of sinusal mucosa: an in vitro study. Amer. J. Physiol. 271, L349-L358, 1996.

Alberty, J. The effect of antiallergic intranasal formulations on ciliary beat fequency of human nasal epithelium in vitro. Allergy 53, 986-989, 1998.

Su, X.Y., Mattern, C., Haecker, R. and Li Wan Po, A. Does sea-water made isotonic affect ciliary beat frequency? Int. J. of Pharmaceutics 123, 47-51, 1995.

Especially, the nasal compositions of the invention show unexpected and superior properties what the transmucosal ion transport is concerned. The latter can be studied in vitro e.g. by the "voltage clamp technique": In this method, the compositions are applied onto the ciliary surface, and the passive ionic flux is measured in the sense that positive charges flow across the mucosa from ciliary to submucosal side (which contains a Tyrodebicarbonate buffer as a control solution). With the compositions of the invention, a surprisingly increased passive ionic flux is obtained. Therefrom one can conclude that the compositions of the invention will favor the transmucosal hydro-electrical turnover on which is based the secretion and absorption of the mucosal "sol phase" ( comprising water and ions). Therefore in situ, where the mucus is constantly cleared by ciliary transport, the compositions of the invention will - to an unexpectedly great extent - stimulate the hydroelectrolyte secretion by the respiratory epithelium.

Moreover, consumer research studies show that the nasal compositions of the invention, surprisingly, are perceived more moisturizing and less drying than analogous commercially available compositions (e.g. Otrivin® regular).

Thus, it has surprisingly been found that just the pharmaceutical compositions of the present invention result in liquid nasal formulations with such excellent properties as outlined above.

The nasal compositions of the invention can be manufactured in a manner known per se, for example by conventional mixing and dissolution methods in aqueous vehicles.

The following examples illustrate the invention but do not limit it in any way.

Example 1: Nasal drops containing 0.1% (m/V) of Xylometazoline hydrochloride

| | |
|---|---|
| Xylometazoline hydrochloride | 0.10 % |
| Sodium dihydrogen phosphate dihydrate | 0.50 % |
| Disodium phosphate dodecahydrate | 0.17 % |
| Disodium edetate | 0.05 % |
| Benzalkonium chloride | 0.01 % |
| Sorbitol (70% in water, non-crystallizing) | 2.00 % |
| Hydroxypropyl methyl cellulose (viscosity 4000 mPa•s) | 0.50 % |
| Sodium chloride | 0.40 % |
| Purified water | 97.17 % |
| | 100.90% (m/V) |

Manufacturing method (for a batch of 50 liters): Introduce 48.385 kg of purified water into a dissolutor. Heat to 80°C and add under stirring sodium dihydrogen phosphate dihydrate, disodium phosphate dodecahydrate, disodium edetate, sorbitol and sodium chloride. Disperse slowly under stirring the hydroxypropyl methyl cellulose into the solution obtained.

Cool the solution to 25°C. Dissolve separately the benzalkonium chloride in 200g purified water under stirring. Add the benzalkonium chloride solution to the former solution. Add xylometazoline hydrochloride to the solution and dissolve under stirring. Filter solution through a mesh screen (ca. 50 micron).

Example 2: Nasal spray containing 0.05% (m/V) of Oxymetazoline hydrochloride

The composition and manufacturing method is the same as in Example 1, with the exception that 0.05% oxymetazoline hydrochloride is used (instead of 0.10% xylometazoline hydrochloride) and the content of purified water is 97.22% (instead of 97.17%). The solution is filled into a squeeze bottle fitted with a nosepiece and a protection cap.

**Claims**

**1.** A liquid nasal pharmaceutical composition which comprises

  (a) one or more active substances suitable for nasal administration and selected from the group consisting of vasoconstrictors, antiallergic agents and corticosteroids,
  (b) sorbitol;
  (c) a water-soluble $C_1$-$C_4$-alkyl-cellulose derivative;
  (d) a vehicle which is present in an amount of at least 90% (m/V) of the total composition, and which is selected from water and mixtures of water with propylene glycol, water with glycerol and water with both propylene glycol and glycerol, whereby in all said mixtures water is present in an amount of at least 95% (m/V); and
  (e) optionally one or more nasally acceptable excipients.

**2.** A pharmaceutical composition according to claim 1, wherein the active substance (a) is selected from the group consisting of vasoconstrictors and antiallergic agents.

**3.** A pharmaceutical composition according to claim 1, wherein the active substances (a) represent a combination of a vasoconstrictor and an antiallergic agent.

4. A pharmaceutical composition according to claim 1, wherein the active substance (a) is selected from the group of vasoconstrictors consisting of xylometazoline, naphazoline, fenoxazoline, oxymetazoline, tetrahydrozoline, tramazoline, phenylephrine, ephedrine, epinephrine, and a nasally acceptable salt of any of these compounds.

5. A pharmaceutical composition according to claim 1, wherein the active substance (a) is selected from the group of vasoconstrictors consisting of xylometazoline, oxymetazoline and a nasally acceptable salt of any of these two compounds.

6. A pharmaceutical composition according to any one of claims 1 to 5, wherein sorbitol (b) is present in an amount of from 0.5 up to 5% (m/V) of the total composition.

7. A pharmaceutical composition according to any one of claims 1 to 6, wherein the water-soluble $C_1$-$C_4$-alkyl-cellulose derivative is selected from the group consisting of methyl cellulose and (hydroxy or carboxy)-substituted $C_1$-$C_4$-alkyl-celluloses.

8. A pharmaceutical composition according to any one of claims 1 to 6, wherein the water-soluble $C_1$-$C_4$-alkyl-cellulose derivative (c) is hydroxypropyl methyl cellulose.

9. A pharmaceutical composition according to any one of claims 1 to 8, wherein the water-soluble $C_1$-$C_4$-alkyl-cellulose derivative (c) is present in an amount of from 0.3 up to 1% (m/V) of the total composition.

10. A pharmaceutical composition according to any one of claims 1 to 9, wherein the vehicle (d) is water.

11. A pharmaceutical composition according to any one of claims 1 to 10, which contains as nasally acceptable excipients (e) essentially the following components: sodium dihydrogen phosphate dihydrate and disodium phosphate dodecahydrate as buffering agents, disodium edetate as chelating agent, benzalkonium chloride as preservative, and sodium chloride as isotonicity regulator.

12. A pharmaceutical composition according to any one of claims 1 to 11, which contains as nasally acceptable excipients (e) essentially the following components: 0.3-0.7% sodium dihydrogen phosphate dihydrate and 0.12-0.25% disodium phosphate dodecahydrate as buffering agents, 0.02-0.08% disodium edetate as chelating agent, 0.005-0.02% benzalkonium chloride as preservative, and 0.20-0.60% sodium chloride as isotonicity regulator.

13. A pharmaceutical composition according to any one of claims 1-4 and 6-12, which consists essentially of 0.025-0.2% of one or more vasoconstrictors selected from the group consisting of xylometazoline, naphazoline, fenoxazoline, oxymetazoline, tetrahydrozoline, tramazoline, phenylephrine, ephedrine, epinephrine, and nasally acceptable salts of said compounds; 1.2-1.6% sorbitol, 0.3-0.7% hydroxypropyl methyl cellulose, 0.3-0.7% sodium dihydrogen phosphate dihydrate, 0.12-0.25% disodium phosphate dodecahydrate, 0.02-0.08% disodium edetate, 0.005-0.02% benzalkonium chloride and 0.20-0.60% sodium chloride, the remainder being water.

14. A pharmaceutical composition according to claim 13, wherein the vasoconstrictor selected is xylometazoline hydrochloride.

15. A pharmaceutical composition according to any one of claims 1 to 14, which is in the form of drops, a solution, a spray or a metered-dose spray.

**Patentansprüche**

1. Flüssige, nasale pharmazeutische Zusammensetzung, die umfaßt

a) einen oder mehrere Wirkstoffe, die zur nasalen Verabreichung geeignet sind und ausgewählt sind aus der Gruppe, die besteht aus Vasokonstriktoren, antiallergischen Mitteln und Corticosteroiden,
b) Sorbit,
c) ein wasserlösliches $C_1$-$C_4$ Alkylcellulosederivat,

d) einen Träger, der in einer Menge von mindestens 90 % (G/V) der Gesamtzusammensetzung vorhanden ist und ausgewählt ist aus Wasser und Gemischen aus Wasser mit Propylenglycol, Wasser mit Glycerin und Wasser sowohl mit Propylenglycol als auch Glycerin, wobei in all diesen Gemischen Wasser in einer Menge von mindestens 95 % (G/V) vorhanden ist, und

e) wahlweise einen oder mehrere nasal annehmbare Hilfsstoffe.

2. Pharmazeutische Zusammensetzung nach Anspruch 1, worin der Wirkstoff (a) aus der Gruppe ausgewählt ist, die besteht aus Vasokonstriktoren und antiallergischen Mitteln.

3. Pharmazeutische Zusammensetzung nach Anspruch 1, worin die Wirkstoffe (a) eine Kombination aus einem Vasokonstriktor und einem antiallergischen Mittel sind.

4. Pharmazeutische Zusammensetzung nach Anspruch 1, worin der Wirkstoff (a) aus der Gruppe der Vasokonstriktoren ausgewählt ist, die besteht aus Xylometazolin, Naphazolin, Fenoxazolin, Oxymetazolin, Tetrahydrozolin, Tramazolin, Phenylephrin, Ephedrin, Epinephrin und einem nasal annehmbaren Salz jeder dieser Verbindungen.

5. Pharmazeutische Zusammensetzung nach Anspruch 1, worin der Wirkstoff (a) aus der Gruppe der Vasokonstriktoren ausgewählt ist, die besteht aus Xylometazolin, Oxymetazolin und einem nasal annehmbaren Salz jeder dieser zwei Verbindungen..

6. Pharmazeutische Zusammensetzung nach einem der Ansprüche 1 bis 5, worin Sorbit (b) in einer Menge von 0,5 bis zu 5 % (G/V) der Gesamtzusammensetzung vorhanden ist.

7. Pharmazeutische Zusamensetzung nach einem der Ansprüche 1 bis 6, worin das wasserlösliche $C_1$-$C_4$ Alkylcellulosederivat aus der Gruppe ausgewählt ist, die besteht aus Methylcellulose und hydroxy- oder carboxysubstituierten $C_1$-$C_4$ Alkylcellulosen.

8. Pharmazeutische Zusammensetzung nach einem der Ansprüche 1 bis 6, worin das wasserlösliche $C_1$-$C_4$ Alkylcellulosederivat (c) Hydroxypropylmethylcellulose ist.

9. Pharmazeutische Zusammensetzung nach einem der Ansprüche 1 bis 8, worin das wasserlösliche $C_1$-$C_4$ Alkylcellulosederivat (c) in einer Menge von 0,3 bis zu 1 % (G/V) der Gesamtzusammensetzung vorhanden ist.

10. Pharmazeutische Zusammensetzung nach einem der Ansprüche 1 bis 9, worin der Träger (d) Wasser ist.

11. Pharmazeutische Zusammensetzung nach einem der Ansprüche 1 bis 10, die als nasal annehmbare Hilfsstoffe (e) im wesentlichen die folgenden Komponenten enthält: Natriumdihydrogenphosphatdihydrat und Dinatriumphosphatdodecahydrat als puffernde Mittel, Dinatriumedetat als Chelatmittel, Benzalkoniumchlorid als Konservierungsstoff und Natriumchlorid als Isotonizitätsregulator.

12. Pharmazeutische Zusammensetzung nach einem der Ansprüche 1 bis 11, die als nasal annehmbare Hilfsstoffe (e) im wesentlichen die folgenden Komponenten enthält: 0,3-0,7 % Natriumdihydrogenphosphatdihydrat und 0,12-0,25 % Dinatriumphosphatdodecahydrat als puffernde Mittel, 0,02-0,08 % Dinatriumedetat als Chelatmittel, 0,005-0,02 % Benzalkoniumchlorid als Konservierungsstoff und 0,20-0,60 % Natriumchlorid als Isotonizitätsregulator.

13. Pharmazeutische Zusammensetzung nach einem der Ansprüche 1-4 und 6-12, die im wesentlichen besteht aus 0,025-0,2 % von einem oder mehreren Vasokonstriktoren, ausgewählt aus der Gruppe, die besteht aus Xylometazolin, Naphazolin, Fenoxazolin, Oxymetazolin, Tetrahydrozolin, Tramazolin, Phenylephrin, Ephedrin, Epinephrin und nasal annehmbaren Salzen dieser Verbindungen, 1,2-1,6 % Sorbit, 0,3-0,7 % Hydroxypropylmethylcellulose, 0,3-0,7 % Natriumdihydrogenphosphatdihydrat, 0,12-0,25 % Dinatriumphosphatdodecahydrat, 0,02-0,08 % Dinatriumedetat, 0,005-0,02 % Benzalkoniumchlorid und 0,20-0,60 % Natriumchlorid, wobei der Rest Wasser ist.

14. Pharmazeutische Zusammensetzung nach Anspruch 13, worin der ausgewählte Vasokonstriktor Xylometazolinhydrochlorid ist.

15. Pharmazeutische Zusammensetzung nach einem der Ansprüche 1 bis 14, die in Form von Tropfen, einer Lösung, eines Sprays oder eines Dosiersprays vorliegt.

**Revendications**

1. Composition pharmaceutique nasale liquide qui comprend

   (a) une ou plusieurs substances actives appropriées à l'administration nasale et choisies dans le groupe constitué par des vasoconstricteurs, des agents anti-allergiques et des corticostéroïdes,
   (b) du sorbitol ;
   (c) un dérivé d'alkylcellulose en $C_1$ à $C_4$ hydrosoluble ;
   (d) un véhicule qui est présent en une quantité d'au moins 90 % (p/v) de la composition totale, et qui est choisi entre l'eau et des mélanges d'eau et de propylèneglycol, d'eau et de glycérol et d'eau, de propylèneglycol et de glycérol, de l'eau étant ainsi présente dans tous lesdits mélanges en une quantité d'au moins 95 % (p/v); et
   (e) éventuellement un ou plusieurs excipients acceptables pour des applications nasales.

2. Composition pharmaceutique suivant la revendication 1, dans laquelle la substance active (a) est choisie dans le groupe constitué par des vasoconstricteurs et des agents anti-allergiques.

3. Composition pharmaceutique suivant la revendication 1, dans laquelle la substance active (a) représente une association d'un vasoconstricteur et d'un agent anti-allergique.

4. Composition pharmaceutique suivant la revendication 1, dans laquelle la substance active (a) est choisie dans le groupe des vasoconstricteurs, consistant en la xylométazoline, la naphazoline, la fénoxazoline, l'oxymétazoline, la tétrahydrozoline, la tramazoline, la phényléphrine, l'éphédrine, l'épinéphrine, et un sel acceptable pour des applications nasales de l'un quelconque de ces composés.

5. Composition pharmaceutique suivant la revendication 1, dans laquelle la substance active (a) est choisie dans le groupe des vasoconstricteurs, consistant en la xylométazoline, l'oxymétazoline et un sel acceptable pour des applications nasales de l'un quelconque de ces deux composés.

6. Composition pharmaceutique suivant l'une quelconque des revendications 1 à 5, dans laquelle le sorbitol (b) est présent en une quantité de 0,5 à 5 % (p/v) de la composition totale.

7. Composition pharmaceutique suivant l'une quelconque des revendications 1 à 6, dans laquelle le dérivé d'alkylcellulose en $C_1$ à $C_4$ hydrosoluble est choisi dans le groupe constitué par la méthylcellulose et des alkylcelluloses en $C_1$ à $C_4$ hydroxy- ou carboxy-substituées.

8. Composition pharmaceutique suivant l'une quelconque des revendications 1 à 6, dans laquelle le dérivé d'alkylcellulose en $C_1$ à $C_4$ hydrosoluble (c) est l'hydroxypropylméthylcellulose.

9. Composition pharmaceutique suivant l'une quelconque des revendications 1 à 8, dans laquelle le dérivé d'alkylcellulose en $C_1$ à $C_4$ hydrosoluble (c) est présent en une quantité de 0,3 à 1% (p/v) de la composition totale.

10. Composition pharmaceutique suivant l'une quelconque des revendications 1 à 9, dans laquelle le véhicule (d) est l'eau.

11. Composition pharmaceutique suivant l'une quelconque des revendications 1 à 10, qui contient essentiellement comme excipients acceptables pour des applications nasales (e) les composants suivants : du dihydrogénophosphate de sodium dihydraté et du dodécahydrate de phosphate disodique comme agents tampons, de l'édétate disodique comme agent chélatant, du chlorure de benzalkonium comme conservateur, et du chlorure de sodium comme régulateur de l'isotonicité.

12. Composition pharmaceutique suivant l'une quelconque des revendications 1 à 11, qui contient essentiellement comme excipients acceptables pour des applications nasales (e) les composants suivants : 0,3-0,7 % de dihydrogénophosphate de sodium dihydraté et 0,12-0,25 % de dodécahydrate de phosphate disodique comme agents tampons, 0,02-0,08 % d'édétate disodique comme agent chélatant, 0,005-0,02 % de chlorure de benzalkonium comme conservateur, et 0,20-0,60 % de chlorure de sodium comme régulateur de l'isotonicité.

13. Composition pharmaceutique suivant l'une quelconque des revendications 1-4 et 6-12, qui est essentiellement constituée de 0,025-0,2 % d'un ou plusieurs vasoconstricteurs choisis dans le groupe constitué par la xylométazoline, la naphazoline, la fénoxazoline, l'oxymétazoline, la tétrahydrozoline, la tramazoline, la phényléphrine, l'éphédrine, l'épinéphrine et des sels acceptables pour des applications nasales desdits composés ; 1,2-1,6 % de sorbitol, 0,3-0,7 % d'hydroxypropylméthylcellulose, 0,3-0,7 % de dihydrogénophosphate de sodium dihydraté, 0,12-0,25 % de dodécahydrate de phosphate disodique, 0,02-0,08 % d'édétate disodique, 0,005-0,02 % de chlorure de benzalkonium et 0,20-0,60 % de chlorure de sodium, le reste étant constitué d'eau.

14. Composition pharmaceutique suivant la revendication 13, dans laquelle le vasoconstricteur choisi est le chlorhydrate de xylométazoline.

15. Composition pharmaceutique suivant l'une quelconque des revendications 1 à 14, qui est sous forme de gouttes, d'une solution, d'un produit d'atomisation ou d'un produit d'atomisation à doses mesurées.